# EUROPEAN PATENT APPLICATION

(11) **EP 4 295 693 A1**
(43) Date of publication of application: **27.12.2023**
(21) Application number: 22756416.8
(22) Date of filing: 07.02.2022
(51) Int. Cl.: A23F 3/16, A23F 3/06, A23F 3/14, A23F 3/18

(54) **METHOD FOR PROMOTING ANTIOXIDANT ACTIVITY OF PURPLE TEA LEAVES**

(30) Priority: 18.02.2021 KR 20210021691
(71) Applicant: Cleanbeauty Inc., Seoul 04995 (KR)
(72) Inventor: LEE, Jin Min, Seoul 06515 (KR)
(74) Representative: FRKelly
(86) International application number: PCT/KR2022/001840
(87) International publication number: WO 2022/177215

(57) **Abstract**

The present invention relates to a method for enhancing the antioxidant activity of purple tea leaves, and a use thereof, the method comprising a step of roasting purple tea leaves at a temperature of 140-180**°C**, and then performing extraction with water or ultrasonic waves. An extraction method at 100**°C**provides higher antioxidant activity than ultrasonic extraction at 60**°C**, and the antioxidant activity of purple tea having passed through roasting at 140°**C**was superior to that of the original purple tea.

## Description

### [Technical Field]

The present invention relates to a method for enhancing the antioxidant activity of purple tea leaves and use of the same.

### [Background Art]

The skin is the largest organ in the body, always in direct contact with the external environment, and plays a role as a protective film to protect the living body from various stimuli or dry environments and compared to other organs, it is an organ where new cells are actively created and destroyed. In addition, it plays a very important role in protecting the body from physical abrasions, preventing loss of moisture inside the body, protecting from ultraviolet rays generated by the sun, and regulating body temperature.

When such skin is subjected to external stimuli due to various physical factors, external environmental factors, etc., aging such as wrinkle formation, loss of elasticity, and keratinization occurs. Skin aging is largely divided into natural aging (or endogenous aging) and external aging. Natural aging is difficult to control artificially because it is affected by genetic factors, whereas external aging is relatively easy to control artificially because it is influenced by environmental factors..

UV rays are a representative external aging factor, and the most prominent external aging phenomenon is wrinkle formation (Daniell HW, Ann Intern Med, 1971, 75(6), 873; Grove GL et al., J Am Acad Dermatol, 1989, 21 (3),631; Griffiths CE et al., Arch Dermatol, 1992, 128, 347). One of the mechanisms of photoaging caused by ultraviolet light is via the free radical pathway (Harman D, J Gerontol, 1956, 11(3), 298)

Even in normal cells, free radicals and other active oxygen and peroxides are generated to some extent during the metabolic process, but in the body, superoxide dismutase (SOD), catalase, and peroxide are used as defense mechanisms against them. Along with antioxidant enzymes such as oxidase, antioxidant substances such as vitamin C, vitamin E, glutathione, ubiquinone, and uric acid exist to protect themselves. However, when an abnormality occurs in such a biological defense mechanism or when the generation of active oxygen exceeds the capacity of the biological defense system due to various physical and chemical factors, oxidative stress is caused. Therefore, suppressing active oxygen is an important factor in skin aging cosmetics.

For neutralizing these reactive oxygen species, antioxidants such as tocopherols, polyphenols, Coenzyme Q 10, BHT, and BHA are widely used, but most of them are synthetic products, and their safety is problematic when applied to human skin for a long time, so their use is limited. Accordingly, many studies are being conducted to develop a natural antioxidant of plant origin, which has high antioxidant effect, is safe for human skin, and is economical..

Since many plant species contain a variety of polyphenols with excellent plant protection effects, numerous plant extracts such as green tea, moth bark, and Scutellaria baicalensis GEORGE are widely used in cosmetics as antioxidants.

Natural antioxidants known to date include tocopherols, flavonoids, gossypol, sesamol, oryzanol, and vitamin C.

On the other hand, purple tea, like all other teas, comes from the plant Camellia Sinensis. Tea's purple color is caused by a unique genetic mutation that produces anthocyanins, the same powerful antioxidants found in blueberries.

The original wild mutant was found in small quantities in China. However, a public/private partnership has emerged in Kenya to isolate this mutation and mass-produce purple tea. It grows at elevations between 4,500 and 7,500 feet, mostly in the Nandi Hills region of Kenya. Higher altitudes near the equator result in higher UV impact and cause plants to produce very high levels of antioxidants to protect leaves from damage.

Anthocyanins have many medicinal properties and are known to be particularly beneficial for cardiovascular diseases. These antioxidants are known to provide anticancer effects, improve eyesight, and aid in cholesterol and blood sugar metabolism. At the same time, the caffeine content is lower than black tea. Purple tea also contains higher amounts of another antioxidant, polyphenols.

Like green tea, purple tea is high in another form of antioxidant known as catechins, especially EGCG, a powerful antioxidant found in green tea. These neuroprotective antioxidants penetrate the blood-brain barrier and significantly enhanced brain antioxidant capacity in a study in rats.

Purple tea contains a special type of polyphenol called GHG that is not found in other teas or foods. Early research suggests that this substance reduces body fat mass and thickness.

### [Prior Patent Literature]

Republic of Korea Patent Publication No. 10-2019-0051680

### [Disclosure]

### [Technical Problem]

The present invention has been made in response to the above needs, and an object of the present invention is to provide a method for enhancing antioxidants of purple tea leaves.

### [Technical Solution]

To achieve the above object, the present invention provides a method for increasing the antioxidant activity of purple tea leaves, which comprises the step of roasting purple tea leaves at a temperature range of 140 **°C** to 180 **°C** and then extracting them with water or ultrasonic waves.

In one embodiment of the present invention, the roasting temperature is preferably 140 **°C**, but is not limited thereto.

In another embodiment of the present invention, the water extraction temperature is preferably 100 **°C**, but is not limited thereto.

In another embodiment of the present invention, the ultrasonic extraction temperature is preferably 60 **°C**, but is not limited thereto.

In addition, the present invention provides an antioxidant composition comprising the extract extracted by the method of the present invention as an active ingredient.

In one embodiment of the present invention, the composition is preferably a food, cosmetic or pharmaceutical composition, but is not limited thereto.

Cosmetics comprising the extract according to an embodiment of the present invention are skin and body care cosmetics, and can be used in various products such as essence, ampoule, soap, tonic and body essence, emulsion, lotion, cream (oil-in-water type, water-in-oil type, multi-phase), solution, suspensions (anhydrous and aqueous), anhydrous products (oil and glycol-based), gels, powders, etc. and the extract is included in 0.05 to 100 wt% based on the total cosmetic composition, and in the case of an ampoule formulation, it may consist of 100% of the extract.

Cosmetics may comprise carriers acceptable in cosmetic formulations in addition to the extract of the present invention. Alcohols, oils, surfactants, fatty acids, silicone oils, preservatives, humectants, humectants, viscosity modifiers, emulsions, stabilizers, sunscreens, coloring agents, fragrances, diluents, and the like may be exemplified as the carrier.

Specific compounds or compositions that can be used as the alcohol, oil, surfactant, fatty acid, silicone oil, preservative, humectant, humectant, viscosity modifier, emulsion, stabilizer, sunscreen, coloring agent, fragrance, diluent, etc. are already known in the art. Therefore, those skilled in the art can select and use an appropriate compound or composition.

Here, to give some examples, alcohols comprise higher alcohols, propylene glycol, 1,3-butylene glycol, glycerin, sorbitol, and water-soluble polyhydric alcohols such as polyethylene glycol. Examples of oils comprise avocado oil, palm oil, beef tallow, jojoba oil, etc., examples of preservatives include ethyl paraben, butyl paraben, etc.; moisturizing agents include hyaluronic acid, chondroitin sulfate, pyrrolidone carboxylate, etc.; diluents include ethanol, isopropanol, etc..

More specifically, when the formulation of a cosmetic is a paste, cream or gel, animal fiber, vegetable fiber, wax, paraffin, starch, tracanth, cellulose derivative, polyethylene glycol, silicone, bentonite, silica, talc or zinc oxide as a carrier component can be used.

In addition, when the formulation is a powder or spray, lactose, talc, silica, aluminum hydroxide, calcium silicate or polyamide powder may be used as a carrier component, and, particularly, in the case of a spray, a propellant such as dimethyl ether, chlorofluorohydrocarbon, or propane / butane can additionally be included.

In addition, when the formulation is a solution or emulsion, a solvent, solvating agent or emulsifying agent is used as a carrier component, such as water, ethanol, isopropanol, ethyl carbonate, ethyl acetate, benzyl alcohol, benzyl benzoate, propylene glycol, 1,3 -Butyl glycol oil, glycerol aliphatic ester, polyethylene glycol or fatty acid ester of sorbitan.

In addition, when the formulation is a suspension, a liquid diluent such as water, ethanol or propylene glycol, a suspending agent such as ethoxylated isostearyl alcohol, polyoxyethylene sorbitol ester and polyoxyethylene sorbitan ester, microcrystalline cellulose, aluminum metahydroxide, bentonite, agar or tracanth and the like may be used.

In addition, when the formulation is surfactant-containing cleansing, as carrier components, aliphatic alcohol sulfate, aliphatic alcohol ether sulfate, sulfosuccinic acid monoester, isethionate, imidazolinium derivative, methyl taurate, sarcosinate, fatty acid amide ether sulfate, alkylamidobetaine, aliphatic alcohols, fatty acid glycerides, fatty acid diethanolamides, vegetable oils, linolin derivatives, or ethoxylated glycerol fatty acid esters.

In addition, in the case of a soap comprising an extract according to an embodiment of the present invention, it may be prepared by including the extract in a soap base, and may be prepared by including a skin moisturizer, an emulsifier, a water softener, and the like as additives.

Vegetable oils such as palm oil, palm oil, soybean oil, castor oil, olive oil, and palm kernels, or animal oils such as beef tallow, lard, brisket, and fish oil may be used as the soap base material, and as the skin moisturizer, glycerin, erythritol, polyethylene glycol, Propylene glycol, butylene glycol, pentylene glycol, hexyl glycol, isopropyl myristate, silicone derivatives, aloe vera, sorbitol, etc. may be used. Examples of the emulsifier include natural oils, waxy fatty alcohols, hydrocarbons, natural plant extracts, etc. may be used, and tetrasodium EDTA may be used as the water softening agent.

The soap composition of the present invention may further include an antibacterial agent, a dispersing agent, a foam inhibitor, a solvent, an anti-scaling agent, a corrosion inhibitor, a flavoring agent, a coloring agent, a metal ion sequestering agent, an antioxidant, a preservative, and the like as additives.

Foods comprising extracts according to an embodiment of the invention include health functional foods comprising food additives or extracts comprising the extract as a functional raw material.

As used herein, the term "food composition" is a formulation selected from the group consisting of tablets, pills, powders, granules, powders, capsules and liquid formulations including at least one of carriers, diluents, excipients and additives.

Examples of foods that can be added to the composition of the present invention comprise various foods, powders, granules, tablets, capsules, syrups, beverages, gum, tea, vitamin complexes, health functional foods, and the like. Additives that may be further included in the present invention may be at least one component selected from the group consisting of natural carbohydrates, flavors, nutrients, vitamins, minerals (electrolytes), flavors (synthetic flavors, natural flavors, etc.), colorants, fillers, pectic acid and salts thereof, alginic acid and its salts, organic acids, protective colloidal thickeners, pH regulators, stabilizers, preservatives, antioxidants, glycerin, alcohols, carbonation agents and fruit flesh.

Examples of the natural carbohydrates comprise monosaccharides such as glucose, fructose, and the like; disaccharides such as maltose, sucrose and the like; and polysaccharides such as conventional sugars such as dextrins and cyclodextrins, and sugar alcohols such as xylitol, sorbitol and erythritol. As the flavoring agent, natural flavoring agents (thaumatin, stevia extract (e.g., rebaudioside A, glycyrrhizin, etc.) and synthetic flavoring agents (saccharin, aspartame, etc.) can be advantageously used.

In addition to the above, the composition according to the present invention may comprise various nutrients, vitamins, minerals (electrolytes), flavors such as synthetic flavors and natural flavors, colorants and enhancers, lactic acid and salts thereof, alginic acid and salts thereof, organic acids, and protective colloidal thickener, a pH adjusting agent, a stabilizer, a preservative, glycerin, alcohol, a carbonating agent used in carbonated beverages, and the like.

Specific examples of the carrier, excipient, diluent, and additive is preferably at least one selected from the group consisting of, but are not limited to, lactose, dextrose, sucrose, sorbitol, mannitol, erythritol, starch, acacia gum, calcium phosphate, alginate, gelatin, calcium phosphate, calcium silicate, microcrystalline cellulose, polyvinylpyrrolidone, cellulose, polyvinylpyrrolidone, methylcellulose, water, sugar syrup, methyl hydroxy benzoate, propyl hydroxy benzoate, talc, magnesium stearate and mineral oil.

The food composition may be used together with ingredients of other conventional food compositions and may be appropriately used according to conventional methods.

In general, when preparing a composition for food, it may be added in an amount of 0.01 to 10 parts by weight, preferably 0.05 to 1 part by weight, based on the raw material of the active ingredient.

However, in the case of long-term intake for the purpose of health and hygiene or health control, the amount may be less than the above range.

In addition to the above, the food composition of the present invention may further include a food-acceptable additive.

In the present invention, the antioxidant effect was confirmed through the extraction method of purple tea leaves and the roasting process, which is a pretreatment process.

### [Advantageous Effects]

As can be seen through the present invention, it was confirmed that the extraction method at 100 **°C**has higher antioxidant activity than the ultrasonic extraction at 60 **°C**, and the antioxidant activity is improved compared to the original purple tea when subjected to the roasting process at 140**°C**.

### [Description of Drawings]

Figure 1 is a picture showing the antioxidant effect of 100 **°C**water-extracted purple tea leaf extract,
Figure 2 is a picture showing the antioxidant effect of 60°**C**ultrasonic water extraction purple tea leaf extract, and
Figure 3 is a picture showing the antioxidant effect of purple tea extract according to the roasting temperature and extraction method.

### [Mode for Invention]

Hereinafter, the present invention will be described in more detail by the following examples. However, the following examples are described with the intention of illustrating the present invention, and the scope of the present invention is not to be construed as being limited by the following examples.

### [Example]

### Example 1: Antioxidant activity analysis according to extraction method

Purple tea leaves (Kangaita (KTDA) purple tea; KTDA Farmers Building Moi Avenue, Nairobi P.O. Box 30213 - 00100 Nairobi, Kenya) were purchased and extracted by two methods.

First, 5 g of purple tea leaves were put in 50 ml of 100**°C** water and extracted by brewing for 5 minutes.

Second, the raw material of purple tea leaves was ultrasonically extracted at 60°C for 2 hours.

As a pre-processing process, the condition for roasting purple tea leaves is to stir 240g of purple tea leaves with a spatula for 4 minutes at 140**°C**, 160**°C**, 180**°C** in an electric pan (Daewon Appliances multipurpose steamer fan DW-1200), cool for 30 minutes, and the dust was filtered using a fine sieve.

The roasted purple tea leaves were extracted by two extraction methods,
After extraction, the extract was centrifuged at 13000 rpm and 4**°C** for 20 minutes, and then the supernatant was passed through a 0.2 µmι filter twice and then lyophilized and diluted to each test concentration and used.

First, to compare the antioxidant activity of purple tea leaves according to the extraction method, purple tea leaves were extracted using 100**°C**extraction and 60**°C**ultrasonic extraction methods, and then ABTS and DPPH assays were performed using various concentrations of the extract to analyze the antioxidant activity.

When DPPH, a purple compound that shows light absorption at 520 nm in the form of a free radical, reacts with a substance with antioxidant activity, it causes a structural change and turns yellow without light absorption at 520 nm.

ABTS is an assay that it reacts with phenolic compounds to form cations and change color and can calculate antioxidant activity by measuring in the range of 600-750nm.

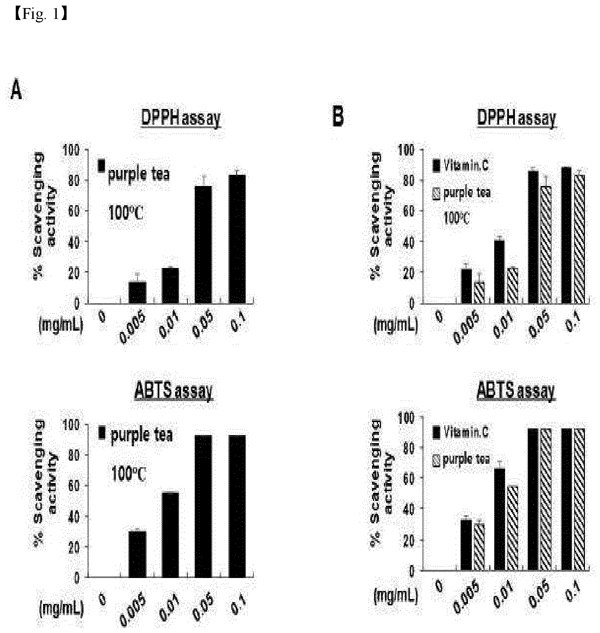

As a result, as can be seen in FIG. 1 and Table 1, 0.05 mg/ml of the extract extracted from purple tea leaves at 100 **°C**had a radical reduction effect of 76.1 ± 6.9% in the DPPH assay and 92.0 ± 0.3% in the ABTS assay.

This is an efficacy corresponding to 93.8% of the radical reduction effect of vitamin C at the same concentration.

At a concentration of 0.01 mg/ml, the radical reduction effect was 22.2±2.0% in the DPPH assay and 54.8±0.1% in the ABTS assay (see FIG. 1 and Table 1).

**[Table 1]**

| DPPH | | | | | | | ABTS | | | | |
|---|---|---|---|---|---|---|---|---|---|---|---|
| Extract conc.(mg/ml) | | 0.1 | 0 .05 | 0.01 | 0.0 05 | 0 | 0.1 | 0.05 | 0.01 | 0.005 | 0 |
| Radical scavenging activity (%) | Purple tea | 83 .6 ± 3.3 | 76.1 ± 6.9 | 22.2 ± 2.0 | 13.2 ± 53 | 0 | 92.2 ± 0.2 | 92.0 ± 0.3 | 54.8 ± 0 .1 | 29.6 ± 2.8 | 0 |
| | Vitamin C | 88.1 ± 0.6 | 86.4 ± 1.8 | 41.2 ± 2.6 | 22.4 ± 3.3 | 0 | 92.4 ± 0.05 | 92.4 ± 0.009 | 66.4 ± 4.4 | 32.9 ± 2.9 | 0 |

Table 1 is a table showing the antioxidant effect of purple tea leaf extract extracted with 100**°C**water.

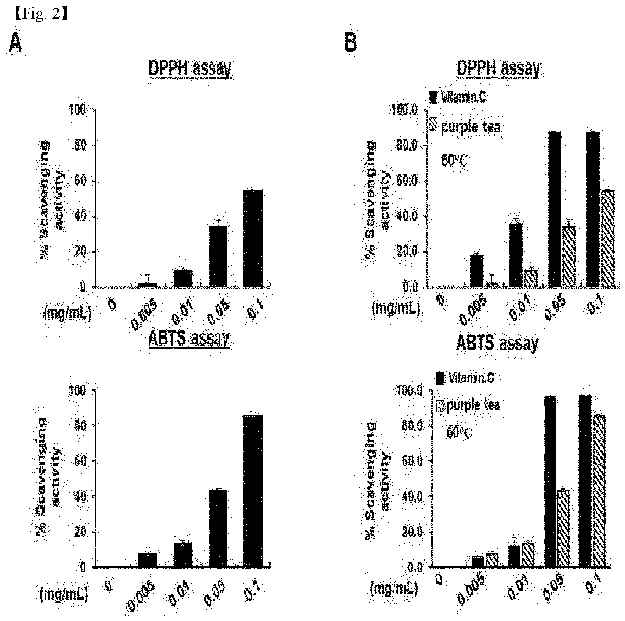

On the other hand, as can be seen in Figure 2 and Table 2, as a result of confirming the antioxidant activity of the purple tea leaf extract extracted by ultrasonication at 60**°C**, at 0.1mg/ml, there was a radical reduction effect of 54.3±0.9% in DPPH assay and 85.3±0.1% in ABTS assay, and in the case of 0.05mg/ml concentration, the radical reduction effect was 33.7±3.5% in the DPPH assay and 44.0±0.4% in the ABTS assay.

This corresponds to 44.2% compared to the radical reduction effect of vitamin C at the same concentration, and the radical reduction effect of the extract was lower than that of the extract extracted at 100 **°C**(see FIG. 2 and Table 2).

**[Table 2]**

| DPPH | | | | | | ABTS | | | | | |
|---|---|---|---|---|---|---|---|---|---|---|---|
| Extractconc. (mg/ml ) | 0.1 | 0.05 | 0.01 | 0.005 | 0 | 0.1 | 0.05 | 0.01 | 0.005 | 0 | |
| Radical scavengingactivity(%) | Purpletea | 54.3 ± 0.9 | 33.7 ± 3.5 | 9.6 ± 1.4 | 1.9 ± 4.9 | 0.0 | 85.3 ± 0.1 | 44. 0 ± 0.4 | 13.5 ± 1.0 | 7.7 ± 1.0 | .0 |
| | Vitamin C | 78.4 ± 0.8 | 8.72 ± 0.7 | 35.7 ± 3.0 | 17.6 ± 1.3 | . 0 | 97. 3 ± 0.1 | 95.9 ± 1.0 | 11.7 ± 4.9 | 5.5 ± 0.8 | .0 |

Table 2 is a table showing the antioxidant effect of purple tea leaf extract extracted with ultrasonic water at 60**°C**

Therefore, the radical reduction effect of the same concentration of purple tea leaves extracted using the 100**°C** extraction method and the 60**°C** ultrasonic extraction method was found to be significantly higher in the radical reduction effect of the 100**°C** extraction method at all concentrations tested in both the DPPH assay and the ABTS assay (see Table 3).

**[Table 3]**

| | DPPH | | | | | | | ABTS | | | |
|---|---|---|---|---|---|---|---|---|---|---|---|
| Extract conc.( mg/ml ) | | 0.1 | 0.05 | 0.01 | 0.005 | 0 | 0.1 | 0.05 | 0.01 | 0.005 | 0 |
| Radicalscavengingactivity (%) | 100 **°C** extraction | 83.6 ± 3.3 * * | 76.1 ± 6.9 * * | 22.2 ± 2.0 * * | 13.2 ± 5.3 * * | 0.0 | 92.2 ± 0.2 * * | 92.0 ± 0.3 * * | 54.8 ± 0.1 * * | 29.6 ± 2.8 * * | 0.0 |
| | 60 **°C** ultrasonicex traction | 54.3 ± 0.9 | 33.7 ± 3.5 | 9.6 ± 1.4 | 1.9 ± 4.9 | 0.0 | 85.3 ± 0.1 | 44.0 ± 0.4 | 13.5 ± 1.0 | 7.7 ± 1.0 | 0.0 |

Table 3 is a table showing the antioxidant effect of purple tea leaf extract according to the extraction method, **p-value <0.001.

### Example 2: Antioxidant activity analysis according to roasting temperature

To confirm the effect according to the roasting temperature, purple tea leaves were extracted from the leaves roasted at 140 **°C**, 160 **°C**, and 180 **°C**using 100**°C**extraction and 60 **°C** ultrasonic extraction methods, and then the radical reduction effect was confirmed.

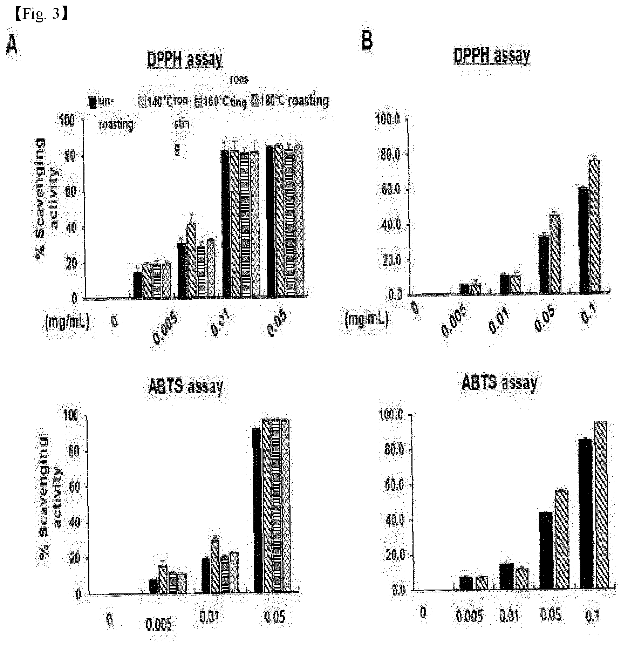

As can be seen in Figure 3A and Table 4, as a result of DPPH assay using the extract extracted using the 100 **°C** water extraction method, purple tea leaf extracts roasted at 140**°C** had a radical reduction effect of 41.2±5.9% and 19.4±0.3% at concentrations of 0.01mg/ml and 0.005mg/ml, respectively, while unroasted purple tea leaf extracts showed 30.8±2.7% and 14.6±2.7% radical reduction effect.

In addition, as a result of the ABTS assay, purple tea leaf extracts roasted at 140**°C** had a radical reduction effect of 29.3±2.0% and 15.9±2.5% at concentrations of 0.01mg/ml and 0.005mg/ml, respectively, while unroasted purple tea leaf extracts showed 19.4±0.9% and 7.6±0.2% radical reduction effect.

The radical reduction effect of the purple tea leaf extract roasted at 140**°C** was significantly higher than that of the unroasted purple tea leaf extract at 142.4% at the 0.01mg/ml concentration and 170.95% at the 0.005mg/ml concentration (See Figure 3A, and Table 4).

**[Table 4]**

| DPPH | | | | | ABTS | | | | |
|---|---|---|---|---|---|---|---|---|---|
| Extract Conc.(mg/ml) | 0.05 | 0.01 | 0.005 | 0 | 0.05 | 0.01 | 0.005 | 0 | |
| Radica 1 scavenging activity (%) | un-ro asting | 81.4 ± 5.2 | 30.8 ± 2.7 | 14.6 ± 27 | 0 | 90.9 ± 0.4 | 19.4 ± 0.9 | 7.6 ± 0.2 | 0.0 |
| | 180 **°C** roasting | 81.0 ± 5.5 | 32.5 ± 0.7 | 18.9 ± 1.4 | 0 | 95.8 ± 0.3 * * | 22.1 ± 0.3 * * | 10.8 ± 0.4 * * | 0. 0 |
| | 160 **°C** roasting | 81.0 ± 3.0 | 28.6 ± 3.3 | 19.3 ± 1.5 | 0 | 96.6 ± 0.0 2 * * | 20.2 ± 0.7 * * | 11.4 ± 0.7 * * | 0. 0 |
| | 140 **°C** roasting | 81.6 ± 6.0 | 41.2 ± 5.9 * | 19.4 ± 0.3 * | 0 | 96.8 ± 0.04 * * | 29.3 ± 2.0 * * | 15.9 ± 2.5 * * | 0.0 |

Table 4 is a table showing the antioxidant effect of the 100 **°C** extraction method according to the roasting temperature, *p-value <0.05, **p-value <0.001

On the other hand, as can be seen in Figure 3B and Table 5, as a result of DPPH assay using the extract extracted using the ultrasonic extraction method at 60 **°C**, purple tea leaf extracts roasted at 140**°C** had a radical reduction effect of 75.1±0.3% and 44.7±1.2% at concentrations of 0.1mg/ml and 0.05mg/ml, respectively, while unroasted purple tea leaf extracts showed 60.0±0.7% and 32.6±0.5% radical reduction effect.

In addition, as a result of the ABTS assay, purple tea leaf extracts roasted at 140**°C** had 93.9±3.1% and 55.7±1.7% radical reduction effects at concentrations of 0.1mg/ml and 0.05mg/ml, respectively, while unroasted purple tea leaf extracts showed 84.7±1.2% and 43.2±2.1% radical reduction effect.

This shows that the radical reduction effect of the purple tea leaf extract roasted at 140 **°C**was significantly higher than that of the unroasted purple tea leaf extract by 118% at the 0.1mg/ml concentration and by 133% at the 0.05mg/ml concentration (Fig. 3B and Table 5).

**[Table 4]**

| DPPH | | | | | | | ABTS | | | | | |
|---|---|---|---|---|---|---|---|---|---|---|---|---|
| Extract Conc. (mg/ml) | 0.1 | 0.05 | 0.01 | 0.005 | 0 | 0.1 | 0.05 | 0.01 | 0.00 5 | 0 | | |
| Radicalscavengingactivity (%) | 6 0 °C ultrasonicextraction | unroasting | 60.0 ± 0.7 | 32.6 ± 0.5 | 10.5 ± 1.1 | 5.4 ± 0.7 | 0.0 | 84.7 ± 1.2 | 43.2 ± 2.1 | 14.6 ± 1.4 | 7.3 ± 0.6 | 0.0 |
| | | 1 40 **°C** roasting | 75.1 ± 0.3 * * | 44.7 ± 1.2 * * | 10.4 ± 1.0 | 5.3 ± 0.9 | 0.0 | 93.9 ± 3.1 * * | 55.7 ± 1.7 * * | 11.6 ± 2.1 * | 6.6 ± 2.7 | 0.0 |

Table 5 is a table showing the antioxidant effect of purple tea roasted at 140 **°C**by ultrasonic extraction at 60 **°C**, *p-value <0.05, **p-value <0.001.

Based on the above results, it was confirmed that the extraction method at 100 **°C**had higher antioxidant activity than the ultrasonic extraction at 60 **°C**, and that the antioxidant activity was improved compared to the original purple tea when roasted at 140 **°C**

## Claims

1. A method of increasing the antioxidant activity of purple tea leaves comprising the step of roasting purple tea leaves at a temperature range of 140 **°C**to 180 **°C**and then extracting them with water or ultrasonic waves.

2. The method of claim 1, wherein the roasting temperature is 140**°C**

3. The method of claim 1, wherein the water extraction temperature is 100**°C**

4. The method of claim 1, wherein the ultrasonic extraction temperature is 60**°C**

5. An antioxidant composition comprising an extract extracted by the method of any one of claims 1 to 4 as an active ingredient.

6. The composition according to claim 5, wherein the composition is a food, cosmetic or pharmaceutical composition.
